# EUROPÄISCHE PATENTSCHRIFT

(11) **EP 2 180 947 B1**
(45) Veröffentlichungstag und Bekanntmachung des Hinweises auf die Patenterteilung: **23.04.2014**
(21) Anmeldenummer: 08774833.1
(22) Anmeldetag: 07.07.2008
(51) Int. Cl.: B01J 23/02, B01J 23/26, B01J 23/72, B01J 23/75, B01J 23/755, B01J 23/86, B01J 37/02, C07C 29/14, C07C 31/125

(54) **HYDRIERKATALYSATOR UND VERFAHREN ZUR HERSTELLUNG VON ALKOHOLEN DURCH HYDRIERUNG VON CARBONYLVERBINDUNGEN**
HYDROGENATION CATALYST AND METHOD FOR THE PRODUCTION OF ALCOHOLS BY HYDROGENATING CARBONYL COMPOUNDS
CATALYSEUR D'HYDROGÉNATION ET PROCÉDÉ DE PRODUCTION D'ALCOOLS PAR HYDROGÉNATION DE COMPOSÉS CARBONYLE

(30) Priorität: 31.08.2007 DE 102007041380
(43) Veröffentlichungstag der Anmeldung: 05.05.2010
(73) Patentinhaber: Evonik Degussa GmbH, 45128 Essen (DE)
(72) Erfinder: KAIZIK, Alfred, 45772 Marl (DE); QUANDT, Thomas, 45772 Marl (DE); LÜKEN, Hans-Gerd, 45770 Marl (DE); BÜSCHKEN, Wilfried, 45721 Haltern am See (DE)
(86) Internationale Anmeldenummer: PCT/EP2008/058780
(87) Internationale Veröffentlichungsnummer: WO 2009/027135

(56) Entgegenhaltungen:
- EP-A- 0 604 840
- EP-A- 1 678 828
- EP-A2- 1 676 828
- WO-A-2004/009526
- DE-A1-102004 055 189
- US-A- 2 795 600
- US-A- 3 781 406
- US-A- 3 894 140
- US-A- 4 666 879
- US-A- 5 124 295

## Beschreibung

Die Erfindung betrifft ein Verfahren zur Herstellung von Alkoholen durch Hydrierung von Carbonylverbindungen, insbesondere von Aldehyden.

Alkohole können durch katalytische Hydrierung von Carbonylverbindungen, die beispielsweise durch Hydroformylierung von Olefinen erhalten worden sind, hergestellt werden. Große Mengen von Alkoholen werden als Lösungsmittel und als Zwischenprodukte für die Herstellung vieler organischer Verbindungen verwendet. Wichtige Folgeprodukte von Alkoholen sind Weichmacher und Detergentien.

Es ist bekannt, Carbonylverbindungen, insbesondere Aldehyde, katalytisch mit Wasserstoff zu Alkoholen zu reduzieren. Dabei werden häufig Katalysatoren eingesetzt, die mindestens ein Metall aus den Gruppen 1 b, 2b, 6b, 7b, und/oder 8 des Periodensystems der Elemente enthalten. Die Hydrierung von Aldehyden kann kontinuierlich oder diskontinuierlich mit pulverförmigen oder stückigen Katalysatoren in der Gas- oder Flüssigphase durchgeführt werden.

Für die technische Herstellung von Alkoholen durch Hydrierung von Aldehyden aus dem Oxo-Prozess (Hydroformylierung von Olefinen) werden, vor allem bei Großprodukten, kontinuierliche Verfahren mit im Festbett angeordneten Katalysatoren in der Gas- oder Flüssigphase angewendet.

Im Vergleich zur Gasphasenhydrierung weist die Flüssigphasenhydrierung die günstigere Energiebilanz und die höhere Raum-Zeit-Ausbeute auf. Mit steigender Molmasse des zu hydrierenden Aldehyds, d. h. mit steigenden Siedepunkten, nimmt der Vorteil der günstigeren Energiebilanz zu. Höhere Aldehyde mit mehr als 7 Kohlenstoffatomen werden demnach vorzugsweise in der Flüssigphase hydriert.

Die Hydrierung in der Flüssigphase hat allerdings den Nachteil, dass aufgrund der hohen Konzentrationen sowohl an Aldehyden als auch an Alkoholen die Bildung von Hochsiedern durch Folge- und Nebenreaktionen begünstigt wird. So können Aldehyde leichter Aldolreaktionen (Addition und/oder Kondensation) eingehen und mit Alkoholen Halb- oder Vollacetale bilden. Die entstandenen Acetale können unter Abspaltung von Wasser oder Alkohol Enolether bilden, die unter den Reaktionsbedingungen zu den gesättigten Ethern hydriert werden. Diese sekundären Nebenprodukte vermindern somit die Ausbeute. Die als Hochsieder bezeichneten Nebenprodukte können bestenfalls teilweise in nachgeschalteten Anlagen in Wertprodukte, wie Ausgangsaldehyde und Zielalkohole, zurückgespalten werden.

Technische Aldehydgemische, die zur Hydrierung eingesetzt werden, enthalten häufig bereits Hochsieder in unterschiedlicher Konzentration.

Bei der Hydroformylierung von Olefinen in Gegenwart von Kobalt-Katalysatoren werden Rohaldehyde erhalten, die neben den Ameisensäureestern (Formiaten) zusätzlich Aldolprodukte, höhere Ester und Ether sowie Acetale als Hochsieder enthalten. Werden diese Gemische in der Gasphase hydriert, so kann der größte Teil der Hochsieder im Verdampfer abgetrennt und in einem separaten Verfahrensschritt zu Wertprodukten aufgearbeitet werden.

Bei der Flüssigphasenhydrierung dagegen bleiben die Hochsieder im Reaktorzulauf. Sie werden in der Hydrierstufe zum größten Teil hydriert, sodass aus ihnen kein Wertprodukt mehr gewonnen werden kann.

In US 5,059,710 wird die Ausbeute an Alkoholen durch Hydrierung von Rohaldehyden dadurch erhöht, dass in einer der Hydrierung vorgeschalteten Verfahrensstufe ein Teil der Hochsieder bei erhöhter Temperatur mit Wasser zu Aldehyden oder Alkoholen zurückgespalten wird. Hydrolyse und Hydrierung sind daher getrennte Verfahrensstufen, wobei über den Wassergehalt des zu hydrierenden Gemischs keine Aussage gemacht wird.

Ein ähnliches Verfahren wird in US 4,401,834 offenbart. Auch hier erfolgt die Spaltung von Hochsiedern in Gegenwart von Wasser vor dem eigentlichen Hydrierungsschritt.

In GB 2 142 010 wird ein Verfahren zur Hydrierung von rohen Aldehyden mit 6 bis 20 C-Atomen, die Hochsieder und geringe Mengen an Schwefelverbindungen enthalten, zu den entsprechenden gesättigten Alkoholen beschrieben. Die Hydrierung erfolgt in zwei hintereinander geschalteten Reaktoren. Der erste Reaktor enthält einen MoS₂/C- und der zweite Reaktor einen Ni/Al₂O₃-Katalysator. Die Hydrierung wird in beiden Reaktoren unter Zusatz von bis zu 10 % Wasserdampf, bezogen auf den Eduktstrom, im Temperaturbereich 180 bis 260 °C und einem Wasserstoffpartialdruck von 150 bis 210 bar mit großem Wasserstoffüberschuss durchgeführt. Dieser ist nach den Beispielen so groß, dass sich das zugesetzte Wasser praktisch nur in der Gasphase befindet. Das Ziel dieses Verfahrens liegt in der Zurückdrängung der Bildung von Kohlenwasserstoffen durch Hydrogenolyse der Alkohole. Über eine Zunahme bzw. Abnahme von Hochsiedern und Formiaten bei der Hydrierung werden keine Aussagen gemacht.

In US 2,809,220 wird eine Flüssigphasenhydrierung von Hydroformylierungsgemischen in Gegenwart von Wasser beschrieben. Als Katalysator werden schwefelhaltige Katalysatoren eingesetzt. Die Hydrierung wird im Druckbereich von 105 bis 315 bar und im Temperaturbereich von 204 bis 315 °C in Gegenwart von 1 bis 10 % Wasser, bezogen auf Edukt, durchgeführt. Um das zugesetzte Wasser in der Gasphase zu halten, wird ein großer Überschuss an Wasserstoff (892 bis 3566 Nm³ Wasserstoff je m³ Edukt) eingesetzt. Bezüglich des hohen Wasserstoffüberschusses wird auf die Diskussion von GB 2 142 010 verwiesen. Nachteilig an diesem Verfahren ist weiterhin der hohe spezifische Energieverbrauch.

Ein weiteres Verfahren zur Hydrierung von Hydroformylierungsgemisches offenbart DE 198 42 370. Hier wird beschrieben, wie Hydroformylierungsgemische in der Flüssigphase an Kupfer, Nickel und Chrom enthaltenden Trägerkatalysatoren hydriert werden können. Je nach Herstellverfahren der Hydroformylierungsgemische (Rhodium- oder Kobaltverfahren) enthalten diese Gemische Wasser. Das offenbarte Verfahren ist für die selektive Hydrierung der Aldehyde zu Alkoholen, ohne Hydrierung der in der Hydroformylierung nicht umgesetzten Olefine ausgelegt, d. h. die Hochsieder (vor allem Acetale) werden nicht zu Wertprodukt umgesetzt. Dies ist wirtschaftlich ungünstig und daher verbesserungsfähig.

In DE 100 62 448 wird ein Verfahren zur kontinuierlichen Hydrierung von Reaktionsgemischen aus der Hydroformylierung von Olefinen mit 4 bis 16 C-Atomen in der homogenen Flüssigphase an Festbettkatalysatoren, die mindestens ein Element der achten Nebengruppe des Periodensystems enthalten, wobei die homogene Flüssigphase des Reaktoraustrags noch zwischen 0,05 bis 10 Massen-% Wasser enthält und im stationärem Zustand des Verfahrens 3 bis 50 % mehr Wasserstoff eingespeist wird, als durch die Hydrierung verbraucht wird.
Dieses Verfahren hat den Vorteil, dass der Anteil an Hochsiedern im Hydrieraustrag zu Beginn einer Hydrierperiode bei frischem Kontakt sehr gering ist. Mit zunehmender Laufzeit jedoch nimmt der Anteil an Hochsieder zu und die Ausbeute an Alkoholen sinkt.

In WO 01/87809 wird zur Verringerung der Bildung von Nebenprodukten bei der Hydrierung von Aldehyden dem Zulauf zum Hydrierreaktor eine darin lösliche Menge einer salzartigen Base (M⁺) (Aⁿ⁻) zugesetzt, wobei (M⁺) für ein Alkalimetallion oder Äquivalent eines Erdalkalimetallions und (Aⁿ⁻) für ein Anion einer Säure mit einem pKa-Wert von größer 2 und n für die Wertigkeit des Anions steht. Nachteilig bei diesem Verfahren ist, dass die zugesetzten Salze sich nach der Hydrierung im Hydrieraustrag befinden. Diese Stoffe können daraus nur aufwändig abgetrennt werden und verursachen somit Kosten. Bei der direkten destillativen Aufarbeitung können sich die zugesetzten Salze in der Destillationsanlage ablagern und zu Produktionsstörungen führen. Wenn dies nicht geschieht, gelangen die zugesetzten Salze in den Destillationssumpf. Dieser ist dann wegen seines Salzgehaltes für viele Zwecke nicht verwendbar.

Es bestand daher die Aufgabe, ein Hydrierverfahren zu entwickeln, mit dem Carbonylverbindungen, insbesondere Aldehyde, in hohen Selektivitäten zu den entsprechenden gesättigten Alkoholen hydriert werden, wobei die Selektivität über einen langen Zeitraum nahezu konstant gehalten wird und der Zusatz von schwer aus dem Hydriergut zu entfernenden Stoffen nicht notwendig ist.

Es wurde nun gefunden, dass Carbonylverbindungen, insbesondere Aldehyde, über lange Zeit mit hoher, nahezu konstanter Selektivität zu den entsprechenden Alkoholen hydriert werden können, wenn ein Hydrierkatalysator verwendet wird, der aus einem Trägermaterial und zumindest einem hydrieraktiven Metall besteht, wobei das Trägermaterial auf Titandioxid, Zirkondioxid, Aluminiumoxid, Siliziumoxid oder deren Mischoxide basiert und das hydrieraktive Metall mindestens ein Element der Gruppe Kupfer, Kobalt, Nickel, Chrom ist, und bei dem das Trägermaterial das Element Barium enthält.

Gegenstand der Erfindung ist mithin ein Verfahren zur Herstellung von Alkoholen durch Hydrierung von Carbonylverbindungen, bei dem man die Hydrierung in Gegenwart eines wie vorstehend charakterisierten Hydrierkatalysators durchführt nach Maßgabe des Anspruch 1.

Das erfindungsgemäße Verfahren unter Verwendung des Katalysators weist eine Reihe von unerwarteten Vorteilen auf.

Die Entstehung von Hochsiedern bei der Hydrierung von Aldehyden in der Flüssigphase durch Nebenreaktionen wie beispielsweise Aldoladdition, Aldolkondensation, Acetalbildung oder Etherbildung ist bei Verwendung der erfindungsgemäßen Katalysatoren sehr gering. Die Selektivität für die Alkoholbildung bleibt über einen langen Zeitraum, typisch mehr als 2000 Betriebsstunden, nahezu konstant. Die Aktivität des Katalysators nimmt nur langsam ab. Sie beträgt beispielsweise bei der Hydrierung entsprechender Hydroformylierungsgemische zu Isononanol etwa nach 2000 Betriebsstunden immer noch mehr als 99 % des Wertes nach 50 h (Anteil der Isononanol-Gewichtsanteile in den Hydrierausträgen). Aus dem Katalysatormaterial werden während der Hydrierung keine Stoffe herausgelöst, die in den Hydrieraustrag gelangen könnten. Dadurch bedingt werden bei der destillativen Aufarbeitung salzfreie Fraktionen erhalten, die deren bessere Nutzung erlauben. Wegen der hohen Selektivität kann die Hydriertemperatur erhöht werden, ohne dass eine merkliche Zunahme von Nebenreaktionen eintritt. Dadurch kann die Raum-Zeit-Ausbeute erhöht oder diese bei Rückgang der Katalysatoraktivität konstant gehalten werden, was zu einer längeren Standzeit des Katalysators führt. Die Erhöhung der Hydriertemperatur ermöglicht die bessere Nutzung der Hydrierwärme.

Der Hydrierkatalysator besteht aus einem Trägermaterial, das auf Titandioxid, Zirkondioxid, Aluminiumoxid, Siliziumoxid oder deren Mischoxide basiert und das das Element Barium enthält, und dem auf diesem Trägermaterial aufgebrachten hydrieraktiven Metall, das zumindest ein Element der Gruppe Kupfer, Kobalt, Nickel, Chrom ist.

Als Trägervorstufe können eingesetzt werden Aluminiumoxid, Alumosilikat, Siliziumdioxid, Titandioxid, Zirkondioxid. Eine bevorzugte Trägervorstufe ist Aluminiumoxid, insbesondere γ-Aluminiumoxid.

Das Trägermaterial bzw. die Trägervorstufe weist im Regelfall Poren auf. Bei den eingesetzten Trägermaterialien kann zwischen Mikroporen (Porendurchmesser kleiner 2 nm), Mesoporen (Porendurchmesser 2 bis 50 nm) und Makroporen (Porendurchmesser größer 50 nm) unterschieden werden. Die Porosität der Trägermaterialien kann einheitlich mikroporös, mesoporös oder makroporös sein, es kann aber auch jede beliebige Kombination dieser Porengrößenklassen vorhanden sein. Häufig sind bimodale Porengrößenverteilungen anzutreffen.
Typischerweise ist die Porosität der Trägermaterialien so, dass der mittlere Porendurchmesser etwa 10 bis 30 nm, die BET-Oberfläche etwa 80 bis 300 m²/g und das Porenvolumen (bestimmt mittels Cyclohexanmethode) etwa 0,4 bis 0,9 ml/g beträgt.

Derartige Trägermaterialien bzw. Trägervorstufen sind an sich bekannt und in vielfältiger Ausgestaltung kommerziell erhältlich.

Die Trägervorstufe wird mit einer Bariumverbindung zum fertigen Träger umgesetzt. Im Trägermaterial liegt das Barium in der Oxydationsstufe 2 als Metalloxid, als Salz der Trägervorstufe, als Mischoxid oder gegebenenfalls als eine andere Verbindung vor. Der Gehalt an Bariumverbindung, berechnet als Bariumoxid, liegt, bezogen auf den reduzierten Katalysator, im Bereich von 0,1 bis 2 Massen-%, insbesondere im Bereich von 0,3 bis 0,7 Massen-%.

Auf das so mit Barium modifizierte Trägermaterial wird mindestens ein hydrieraktives Metall aus der Gruppe Kupfer, Chrom, Nickel, Kobalt aufgebracht. Der Katalysator kann eines oder mehrere der hydrieraktiven Metalle enthalten. Vorzugsweise enthält der Katalysator die Metalle Kupfer, Chrom, Nickel. Besonders bevorzugt enthält der Katalysator als hydrieraktives Metall die Kombination der drei Metalle Kupfer, Chrom und Nickel.

Der Gesamtgehalt an hydrieraktiven Metallen liegt, bezogen auf den reduzierten Katalysator, im Bereich von 1 bis 40 Massen-%, insbesondere im Bereich von 5 bis 25 Massen-%, berechnet als Metall.

Das Verfahren zur Herstellung des Hydrierkatalysators erfolgt in der Weise, dass man in einer ersten Stufe auf ein Trägermaterial basierend auf Titandioxid, Zirkondioxid, Aluminiumoxid, Siliziumoxid oder deren Mischoxide eine Lösung aufbringt, die eine Bariumverbindung enthält, und das so behandelte Trägermaterial trocknet und anschließend kalziniert, und in einer zweiten Stufe auf das so behandelte Trägermaterial eine Lösung aufbringt, die zumindest eine Verbindung der Elemente Kupfer, Kobalt, Nickel, Chrom enthält, und das so behandelte Trägermaterial trocknet und anschließend kalziniert.

In der ersten Verfahrensstufe können auf das Trägervormaterial eine oder mehrere Bariumverbindungen aufgebracht werden. Vorzugsweise erfolgt dies durch Aufsprühen einer Lösung auf das Trägervormaterial oder Tränken des Trägervormaterials mit einer Lösung, die eine oder mehrere Bariumverbindungen enthält.

Geeignete Bariumverbindungen sind beispielsweise Bariumacetat, Bariumchlorid(-Hydrat), Bariumhydroxid-Octahydrat, Bariumnitrat, Bariumchlorid-Dihydrat
Eine bevorzugt eingesetzte Verbindung ist Bariumnitrat.

Vorzugsweise werden die Bariumverbindungen als wässrige Lösung aufgebracht.

Die Aufbringung der Bariumverbindung kann in einem Arbeitsgang oder in mehreren Arbeitsgängen erfolgen, wobei die bei den einzelnen Durchgängen verwendeten Lösungen sich hinsichtlich Konzentration und Zusammensetzung unterscheiden können.

Nach dem Aufbringen der Bariumverbindung wird das rohe Trägermaterial im Temperaturbereich von 80 bis 120 °C im Luftstrom vorgetrocknet. Erfolgt die Aufbringung der Bariumverbindung in mehreren Schritten kann nach jedem Schritt getrocknet werden. Nach der Vortrocknung wird das rohe Trägermaterial im Temperaturbereich von 400 bis 650 °C, insbesondere im Bereich von 420 °C bis 550 °C kalziniert.

Nach der Kalzinierung werden auf das Trägermaterial eine oder mehrere der hydrieraktiven Metalle Kupfer, Chrom, Nickel, Kobalt aufgebracht. Die Aufbringung erfolgt analog wie für die Aufbringung der Bariumverbindung beschrieben, nämlich indem das Trägermaterial mit einer Lösung der entsprechenden Metallverbindungen behandelt wird. Es werden bevorzugt wässrige Lösungen von Verbindungen der hydrieraktiven Metalle verwendet.

Zur Herstellung dieser Lösungen können beispielsweise folgende Verbindungen eingesetzt werden:
Kupferformiat, Kupferacetat, Kupferchlorid, Kupfernitrat, Kupfersulfat, Kupferacetylacetonat und die entsprechenden Aquo- und Amminkomplexe dieser Verbindungen;
Kobaltformiat, Kobaltacetat, Kobaltchlorid, Kobaltnitrat, Kobaltsulfat, Kobaltacetylacetonat sowie davon abgeleitete Aquo- und Amminkomplexe;
Nickelformiat, Nickelacetat, Nickelacetylacetonat, Nickelchlorid, Nickelnitrat, Nickelsulfat und davon abgeleitete Aquo- und Amminkomplexe;
Chromformiat, Chromacetat, Chromacetylacetonat, Chromchlorid; Chromnitrat, Chromsulfat und davon abgeleitete Aquo- und Amminkomplexe sowie Ammoniumchromat und Ammoniumdichromat.

Soll der Katalysator mehr als ein hydrieraktives Metall enthalten, so wird zweckmäßigerweise der Träger mit einer gemeinsamen Lösung von Verbindungen der zu kombinierenden Metalle behandelt. Es ist aber auch möglich, entsprechende Lösungen der zu kombinierenden Metalle nacheinander auf den Träger aufzubringen, wobei nach jedem Schritt getrocknet werden kann.

Insbesondere wird der Katalysatorträger mit einer gemeinsamen Lösung von Verbindungen der drei Metalle Kupfer, Chrom und Nickel behandelt.

Nach der Aufbringung der Verbindungen der hydrieraktiven Metalle und Vortrocknung wird der rohe Katalysator im Temperaturbereich von 400 °C bis 650 °C, insbesondere im Temperaturbereich von 420 °C bis 550 °C kalziniert.

Wenn die hydrieraktiven Metalle als Formiate oder Nitrate auf den Träger aufgebracht werden, kann gegebenenfalls auf eine Kalzinierung verzichtet werden.

Die Katalysatoren werden zweckmäßig in einer Form hergestellt, die bei der Hydrierung einen geringen Strömungswiderstand bietet, wie beispielsweise Tabletten, Zylinder, Strangextrudate oder Ringe. Bei der Herstellung des Katalysators wird üblicherweise das Trägervormaterial in entsprechende Form gebracht. Geformtes Trägervormaterial ist auch kommerziell erhältlich.

Das erfindungsgemäße Verfahren zur Herstellung von Alkoholen durch Hydrierung von Carbonylverbindungen erfolgt in an sich bekannter Weise, wobei man aber die Hydrierung in Gegenwart eines wie oben beschriebenen Hydrierkatalysators durchführt.

Im erfindungsgemäßen Verfahren kann die Hydrierung an suspendierten feinteiligen oder geformten, im Festbett angeordneten Katalysatoren kontinuierlich oder diskontinuierlich durchgeführt werden. Die kontinuierliche Hydrierung an einem im Festbett angeordneten Katalysator, bei der sich unter Reaktionsbedingungen die Produkt/Eduktphase hauptsächlich im flüssigen Zustand befindet, wird bevorzugt.

Wird die Hydrierung kontinuierlich an einem im Festbett angeordneten Katalysator durchgeführt, ist es zweckmäßig, den Katalysator vor der Hydrierung in die aktive Form zu überführen. Dies kann durch Reduktion des Katalysators mit wasserstoffhaltigen Gasen nach einem Temperaturprogramm erfolgen. Dabei kann die Reduktion gegebenenfalls in Gegenwart einer flüssigen Phase, die über den Katalysator geleitet wird, durchgeführt werden, wie etwa in DE 199 33 348 beschrieben.

Das erfindungsgemäße Verfahren wird in der Rieselphase oder bevorzugt in der Flüssigphase in Dreiphasenreaktoren im Gleichstrom durchgeführt, wobei der Wasserstoff in an sich bekannter Weise in dem flüssigen Edukt/ProduktStrom fein verteilt wird. Im Interesse einer gleichmäßigen Flüssigkeitsverteilung, einer verbesserten Reaktionswärmeabfuhr und einer hohen Raum-Zeit-Ausbeute werden die Reaktoren vorzugsweise mit hohen Flüssigkeitsbelastungen von 15 bis 120, insbesondere von 25 bis 80 m³ pro m² Querschnitt des leeren Reaktors und Stunde betrieben. Wird ein Reaktor isotherm und im geraden Durchgang betrieben, so kann die spezifische Katalysatorbelastung (LHSV) Werte zwischen 0,1 und 10 h⁻¹ annehmen.

Das erfindungsgemäße Verfahren wird mit Wasserstoff in einem Druckbereich von 5 bis 100 bar, vorzugsweise zwischen 5 und 40 bar, besonders bevorzugt im Bereich von 10 bis 25 bar durchgeführt. Die Hydriertemperaturen liegen zwischen 120 und 220 °C, insbesondere zwischen 140 und 190 °C.

Der für die Hydrierung eingesetzte Wasserstoff kann inerte Gase wie beispielsweise Methan oder Stickstoff enthalten. Vorzugsweise wird Wasserstoff mit einer Reinheit größer als 98 %, insbesondere größer als 99 % eingesetzt.

Für das erfindungsgemäße Verfahren können unterschiedliche Verfahrensvarianten gewählt werden. Es kann adiabatisch, oder praktisch isotherm, d. h. mit einem Temperaturanstieg kleiner 10 °C, ein- oder mehrstufig durchgeführt werden. Im letzteren Falle kann man alle Reaktoren, zweckmäßig Rohrreaktoren, adiabatisch oder praktisch isotherm sowie einen oder mehrere adiabatisch und die anderen praktisch isotherm betreiben. Weiterhin ist es möglich, die Carbonylverbindungen oder Gemische von Carbonylverbindungen in Gegenwart von Wasser im geraden Durchgang oder mit Produktrückführung zu hydrieren.

Mit dem Hydrierkatalysator und dem erfindungsgemäßen Verfahren können grundsätzlich alle Carbonylverbindungen zu entsprechenden Alkoholen hydriert werden. Insbesondere können Aldehyde zu primären Alkoholen, Ketone zu sekundären Alkoholen, α,β-ungesättigte Aldehyde zu gesättigten primären Alkoholen und α,β-ungesättigte Ketone zu gesättigten sekundären Alkoholen hydriert werden. Diese Carbonylverbindungen können weitere funktionelle Gruppen wie beispielsweise Hydroxyl- oder Alkoxygruppen aufweisen. Darüber hinaus können weitere nicht konjugierte olefinische Doppelbindungen vorhanden sein, die abhängig vom Katalysator und von den weiteren Verfahrensbedingungen nicht, partiell oder vollständig hydriert werden können.

Die Hydrierung von α,β-ungesättigten Ketonen oder Aldehyden wird vorzugsweise ohne Zusatz von Wasser und die Hydrierung von nicht konjugierten Ketonen und Aldehyden vorzugsweise unter Zusatz von Wasser, wie beispielsweise in DE 100 62 448 beschrieben, durchgeführt.

Mit Hilfe des erfindungsgemäßen Verfahrens werden bevorzugt Carbonylverbindungen mit 4 bis 25 C-Atomen hydriert, wie insbesondere gesättigte oder ungesättigte Aldehyde oder Ketone mit 4 bis 25 C-Atomen.

Zur Minimierung von Nebenreaktionen und somit Erhöhung der Alkoholausbeute ist es zweckmäßig, die Konzentration an Carbonylverbindungen im Reaktorzulauf zu begrenzen. Insbesondere bei der Hydrierung von Hydroformylierungsgemischen mit 8 bis 17 C-Atomen liegt der Aldehydgehalt im Reaktorzulauf zwischen 1 und 35 Massen-%, insbesondere zwischen 5 und 25 Massen-%. Der gewünschte Konzentrationsbereich kann bei Reaktoren, die in Schlaufenfahrweise betrieben werden, durch die Zirkulationsrate (Mengenverhältnis von rückgeführten Hydrieraustrag zu Edukt) eingestellt werden.

Die im erfindungsgemäßen Verfahren eingesetzten Carbonylverbindungen können auf verschiedene Wege hergestellt sein:
α,β-ungesättigte Ketone können beispielsweise durch Kondensation von zwei Ketonen oder Kondensation von einem Keton mit einem Aldehyd hergestellt werden, wie beispielsweise Oct-3-en-2-on aus n-Pentanal und Aceton;
α,β-ungesättigte Aldehyde können durch Aldolkondensation von Aldehyden hergestellt werden, beispielsweise 2-Ethylhex-2-enal aus n-Butanal,
2-Propylhept-2-enal aus n-Pentanal oder ein Gemisch isomerer Decenale durch Kondensation von mindestens zwei verschiedenen C₅-Aldehyden. Bevorzugt wird ein Decenalgemisch eingesetzt, hergestellt durch Kondensation von C₅-Aldehyden, wie insbesondere Valeraldehyd.

Die im erfindungsgemäßen Verfahren eingesetzten nicht konjugiert ungesättigten Aldehyde werden vorwiegend durch Hydroformylierung hergestellt.

Die Edukte für die Herstellung der Aldehyde bzw. des Reaktionsgemisches durch Hydroformylierung sind Olefine oder Gemische von Olefinen mit 3 bis 24, insbesondere mit 4 bis 16 Kohlenstoffatomen, mit end- oder innenständigen C-C-Doppelbindungen, wie z. B. 1-Buten, 2-Buten, Isobuten, 1- oder 2-Penten, 2-Methylbuten-1, 2-Methylbuten-2, 3-Methylbuten-1, 1-,2- oder 3-Hexen, das bei der Dimerisierung von Propen anfallende C₆-Olefingemisch (Dipropen), Heptene, 2- oder 3-Methyl-1-hexen, Octene, 2-Methylheptene, 3-Methylheptene, 5-Methylhepten-2, 6-Methylhepten-2, 2-Ethylhexen-1, das bei der Dimerisierung von Butenen anfallende Gemisch der isomeren C₈-Olefine (Dibuten), Nonene, 2- oder 3-Methyloctene, das bei der Trimerisierung von Propen anfallende C₉-Olefingemisch (Tripropen), Decene, 2-Ethyl-1-octen, Dodecene, das bei der Tetramerisierung von Propen oder der Trimerisierung von Butenen anfallende C₁₂-Olefingemisch (Tetrapropen oder Tributen), Tetradecene, Pentadecene, Hexadecene, das bei der Tetramerisierung von Butenen anfallende C₁₆-Olefingemisch (Tetrabuten) sowie durch Cooligomerisierung von Olefinen mit unterschiedlicher Anzahl von Kohlenstoffatomen (bevorzugt 2 bis 4) hergestellte Olefingemische, gegebenenfalls nach destillativer Trennung in Fraktionen mit gleicher oder ähnlicher Kettenlänge. Ebenfalls können Olefine oder Olefingemische, die durch Fischer-Tropsch-Synthese erzeugt worden sind sowie Olefine, die durch Oligomerisierung von Ethen erhalten wurden oder Olefine, die über Metathesereaktionen zugänglich sind, eingesetzt werden. Bevorzugte Edukte für die Herstellung der Hydroformylierungsgemische sind C₈-, C₉-, C₁₂-, C₁₅-oder C₁₆-Olefingemische. Besonders bevorzugt werden im Verfahren gemäß der Erfindung Hydroformylierungsgemische, hergestellt aus C₈- oder C₁₂-Olefinen oder C₈- oder C₁₂-Olefingemischen, eingesetzt. Besonders bevorzugt wird der durch Hydroformylierung von Dibuten erhältliche C₉-Aldehyd Isononanal eingesetzt.

Die Olefine werden in üblicher Weise hydroformyliert und ergeben dann die Edukte für das erfindungsgemäße Hydrierverfahren. Man arbeitet in der Regel mit Rhodium- oder Kobaltkatalysatoren sowie mit oder ohne komplexstabilisierende Zusätze, wie organischen Phosphinen oder Phosphiten. Die Temperaturen und Drücke können, je nach Katalysator oder Olefin, in weiten Grenzen variieren. Eine Beschreibung der Hydroformylierung von Olefinen findet sich z. B. bei J. Falbe, New Syntheses with Carbon Monoxide, Springer-Verlag, Heidelberg-New York, 1980, Seite 99 ff. sowie bei Kirk-Othmer, Encyclopedia of Chemical Technology, Band 17, 4. Auflage, John Wiley & Sons, Seiten 902 bis 919 (1996).

Die Reaktionsgemische der Hydroformylierung werden vor dem Einsatz in dem erfindungsgemäßen Verfahren zweckmäßigerweise zunächst vom Katalysator befreit. Wenn ein Kobaltkatalysator verwendet worden ist, kann dies durch Druckentlastung, Oxidation der im Hydroformylierungsgemisch verbliebenen Kobaltcarbonylverbindungen in Gegenwart von Wasser oder wässriger Säure und Abtrennung der wässrigen Phase geschehen. Entkobaltungsverfahren sind gut bekannt, siehe z. B. J. Falbe, a. a. O., Kirk-Othmer, a. a. O., 164, 175, BASF-Verfahren.

Wenn eine Rhodiumverbindung als Hydroformylierungskatalysator eingesetzt wird, kann man sie z. B mittels Dünnschichtverdampfung als Destillationsrückstand abtrennen.

Die vom Hydroformylierungskatalysator befreiten Reaktionsgemische der Kobalt-katalysierten Hydroformylierung enthalten im Allgemeinen 3 bis 40 Massen-%, meistens 5 bis 30 Massen-% Leichtsieder, hauptsächlich nicht umgesetzte Olefine, daneben die entsprechenden gesättigten Kohlenwasserstoffe sowie 0,05 bis 5 Massen-% Wasser, 30 bis 90 Massen-% Aldehyde, 5 bis 60 Massen-% Alkohole, bis zu 10 Massen-% Formiate dieser Alkohole und 3 bis 15 Massen-% Hochsieder.

Es sei jedoch betont, dass das erfindungsgemäße Verfahren auch mit Hydroformylierungsgemischen ausgeführt werden kann, deren Zusammensetzung in dieser oder jener Beziehung nicht diesen Angaben entspricht. So können beispielsweise vor der Hydrierung die Kohlenwasserstoffe (Olefine und Paraffine) vom Hydroformylierungsgemisch abgetrennt werden.

Die nach dem erfindungsgemäßen Verfahren erhaltenen Hydrierausträge werden destillativ aufgearbeitet. Dies geschieht bei Normaldruck oder vermindertem Druck. Bei hoch siedenden Alkoholen wird die Destillation bei vermindertem Druck bevorzugt.

Die folgenden Beispiele sollen die Erfindung weiter erläutern.

### Beispiel 1: Herstellung eines ersten Hydrierkatalysators

Ein kommerzieller Aluminiumoxidträger (Firma Axens) in Form von Extrudaten mit einem Durchmesser von etwa 1,2 mm, einer BET-Oberfläche von etwa 260 m²/g und einem Porenvolumen (bestimmt mittels Cyclohexanmethode) von 0,7 ml/g wurde zunächst zur partiellen Neutralisation azider Zentren mit Natriumverbindungenverbindungen modifiziert. Dazu wurden 500 g der Extrudate in ein Glasrohr gefüllt und dieses für etwa 30 min evakuiert. Anschließend wurde die Imprägnierlösung, eine verdünnte wässrigen Natriumhydroxidlösung (w(NaOH) = 0,24 %), von unten bis über den oberen Rand der Festkörperschüttung gesaugt. Nach einer Einwirkzeit von etwa 15 Minuten wurde die Lösung, die nicht vom Träger aufgenommen worden war, abgelassen. Die getränkten Extrudate wurden zunächst im Luftstrom bei 120 °C getrocknet, anschließend mit 2 K/min auf 450 °C aufgeheizt und bei dieser Temperatur für 6 h kalziniert. Der so hergestellte Katalysatorvorläufer enthielt formal 0,1 Massen % Natrium.
Der mit Natrium modifizierte Aluminiumoxidträger wurde anschließend über Vakuumimprägnierung mit einer ammoniakalischen Nickel-, Kupfer und Chromverbindungen enthaltenden Lösung getränkt. Dazu wurde zunächst in eine Mischung aus Kupfertetrammincarbonat-Lösung (Cu-Gehalt durch elektrogravimetrische Bestimmung: 13,9 Massen-%, NH₃-Gehalt nach Kjeldahl:13,0 Massen %, Dichte bei 20 °C: 1,242 g/cm) und Nickelhexammincarbonat-Lösung (Ni-Gehalt aus Ausgangsverbindung errechnet: 11,2 Massen-%, NH₃-Gehalt nach Kjeldahl: 18,6 Massen-%, Dichte bei 20 °C: 1,29 g/cm³) eine Ammoniumdichromatlösung (errechneter Chrom-Gehalt: 7,1 Massen %) eingerührt. Der aus den Ausgangsverbindungen errechnete Gehalt an Kupfer, Nickel und Chrom der dunkelgrünen Tränklösung betrug 8,1 Massen-% Kupfer, 3,6 Massen-% Nickel und 0,7 Massen-% Chrom. Die Dichte der Lösung betrug 1,26 g/cm³. Zur Vakuumimprägnierung wurden 500 g der Extrudate in ein Glasrohr gefüllt und dieses für etwa 30 min evakuiert. Anschließend wurde die Imprägnierlösung von unten bis über den oberen Rand der Festkörperschüttung gesaugt. Nach einer Einwirkzeit von etwa 15 Minuten wurde die Lösung, die nicht von dem Träger aufgenommen worden war, abgelassen. Die feuchten Pellets wurden zunächst im Luftstrom bei 120 °C getrocknet, anschließend mit 3 K/min auf 450 °C aufgeheizt und bei dieser Temperatur für 10 h kalziniert. Nach der Kalzination enthielt der Katalysator formal: 86 Massen-% Al₂O₃, 6,4 Massen-% Cu, 2,9 Massen-% Ni, 0,6 Massen-% Cr und 0,09 Massen-% Na.

### Beispiel 2: Herstellung eine zweiten Hydrierkatalysators

Ein kommerzieller Aluminiumoxidträger (Firma Axens) in Form von Extrudaten mit einem Durchmesser von etwa 1,2 mm, einer BET-Oberfläche von etwa 260 m²/g und einem Porenvolumen (bestimmt mittels Cyclohexanmethode) von 0,7 ml/g wurde zunächst zur partiellen Neutralisation azider Zentren mit Bariumverbindungen modifiziert. Dazu wurden 500 g der Extrudate in ein Glasrohr gefüllt und dieses für etwa 30 min evakuiert. Anschließend wurde die Imprägnierlösung, eine verdünnte wässrige Bariumnitratlösung (w(Ba) = 0,4 %), von unten bis über den oberen Rand der Festkörperschüttung gesaugt. Nach einer Einwirkzeit von etwa 15 Minuten wurde die Lösung, die nicht vom Träger aufgenommen worden war, abgelassen. Die getränkten Extrudate wurden zunächst im Luftstrom bei 120 °C getrocknet, anschließend mit 2 K/min auf 450 °C aufgeheizt und bei dieser Temperatur für 6 h kalziniert. Der so hergestellte Katalysatorvorläufer enthielt formal 0,32 Massen-% Barium.
Der mit Barium modifizierte Aluminiumoxidträger wurde anschließend über Vakuumimprägnierung mit einer ammoniakalischen Nickel-, Kupfer- und Chromverbindungen enthaltenden Lösung getränkt. Dazu wurde zunächst in eine Mischung aus Kupfertetrammincarbonat-Lösung (Cu-Gehalt durch elektrogravimetrische Bestimmung: 13,9 Massen-%, NH₃ nach Kjeldahl:13,0 Massen-%, Dichte bei 20 °C: 1,29 g/cm) und Nickelhexammincarbonat-Lösung (Ni-Gehalt aus Ausgangsverbindung errechnet: 10,6 Massen-%, NH₃-Gehalt nach Kjeldahl: 18,0 Massen-%, Dichte bei 20 °C: 1,21 g/cm3) eine Ammoniumdichromatlösung (errechneter Chrom-Gehalt: 7,1 Massen-%) eingerührt. Der aus den Ausgangsverbindungen errechnete Gehalt an Kupfer, Nickel und Chrom der dunkelgrünen Tränklösung betrug 7,7 Massen-% Kupfer, 3,5 Massen-% Nickel und 0,8 Massen-% Chrom. Die Dichte der Lösung betrug 1,23 g/cm³. Zur Vakuumimprägnierung wurden 500 g der Extrudate in ein Glasrohr gefüllt und dieses für etwa 30 min evakuiert. Anschließend wurde die Imprägnierlösung von unten bis über den oberen Rand der Festkörperschüttung gesaugt. Nach einer Einwirkzeit von etwa 15 Minuten wurde die Lösung, die nicht von dem Träger aufgenommen worden war, abgelassen. Die feuchten Pellets wurden zunächst im Luftstrom bei 120 °C getrocknet, anschließend mit 3 K/min auf 450 °C aufgeheizt und bei dieser Temperatur für 10 h kalziniert. Nach der Kalzination enthielt der Katalysator formal: 87 Massen-% Al₂O₃; 6,3 Massen % Cu; 2,8 Massen-% Ni; 0,6 Massen-% Cr und 0,3 Massen-% Ba.

### Beispiel 3: C₉-Aldehyd-Hydrierung in der Flüssigphase am im Beispiel 1 hergestellten Katalysator (Vergleich, nicht erfindungsgemäß)

Ein Reaktionsaustrag aus der Kobalt-katalysierten Hydroformylierung von Dibuten mit 60,65 Massen-% C₉-Aldehyd Isononanal wurde in einer Kreislaufapparatur bei 180 °C und 25 bar absolut an 70,2 g (entsprechend 100 ml) Katalysator in der Flüssigphase kontinuierlich hydriert. Es wurden stündlich 0,075 l Edukt bei einem Kreislauf von 45 l/h durchgesetzt. Die Abgasmenge betrug 60 Nl/h. Die Edukt- und Produkt-Analysen sind in Tabelle 1 wiedergegeben. Die Analyse zum Zeitpunkt Null gibt die Zusammensetzung des Eduktes wieder.

**Tabelle 1:**

| Versuchszeit (Stunden) | C₈-KWST (Massen-%) | C₉-al (Massen-%) | Formiat (Massen- %) | C₉-ol (Massen-%) | Hochsieder Massen-%) |
|---|---|---|---|---|---|
| 0 | 6,31 | 60,65 | 3,95 | 27,15 | 1,94 |
| 50 | 6,21 | 0,52 | 0,48 | 90,81 | 1,98 |
| 500 | 6,05 | 0,72 | 0,49 | 89,99 | 2,75 |
| 1000 | 5,98 | 1,04 | 0,49 | 88,80 | 3,68 |
| 1500 | 5,97 | 1,67 | 0,51 | 87,85 | 3,98 |
| 2000 | 5,81 | 1,99 | 0,51 | 87,12 | 4,60 |

Wie aus der Tabelle 1 zu entnehmen, wurden bei der Hydrierung von Isononanal am Standardkatalysator mit der Versuchszeit verstärkt Hochsieder gebildet. Die Restgehalte an C₉-Aldehyd stiegen von 0,52 Massen-% zu Beginn der Hydrierung auf rd. 2 Massen-% nach 2000 Versuchstunden auf. Der Rückgang der Katalysatoraktivität und die Bildung der Hochsieder hatten zur Folge, dass die Ausbeute der Hydrierung zum Wertprodukt Isononanol mit der Betriebszeit gemindert wurde. Die Gehalte an C₉-Alkohol, die zu Beginn der Hydrierung bei etwa. 90,8 Gew.-% gelegen hatte, fielen während 2000 Stunden auf etwa 87,1 Gew.-% ab.

### Beispiel 4: C₉-Aldehyd-Hydrierung am im Beispiel 2 hergestellten Katalysator (erfindungsgemäß):

Ein Reaktionsaustrag aus der Kobalt-katalysierten Hydroformylierung von Dibuten mit 60,34 Gew.-% C₉-Aldehyd Isononanal wurde in einer Kreislaufapparatur bei 180 °C und 25 bar absolut an 69,5 g (entsprechend 99 ml) Katalysator in der Flüssigphase kontinuierlich hydriert. Die Langzeit-Testung wurde unter vergleichbaren Reaktionsbedingungen wie am Standardkatalysator (aus Beispiel 1) im Beispiel 3 durchgeführt. Es wurden stündlich 0,075 l Edukt bei einem Kreislauf von 45 l/h durchgesetzt. Die Abgasmenge betrug 60 Nl/h. Die Edukt- und Produkt-Analysen sind in Tabelle 2 wiedergegeben.

**Tabelle 2:**

| Versuchszeit (Stunden) | C8-KWST (Massen-%) | C9-al (Massen-%) | Formiat (Massen-%) | C9-ol (Massen-%) | Hochsieder Massen-%) |
|---|---|---|---|---|---|
| 0 | 6,57 | 60,34 | 3,15 | 28,07 | 1,87 |
| 50 | 6,13 | 0,62 | 0,45 | 90,85 | 1,95 |
| 500 | 6,11 | 0,73 | 0,36 | 91,01 | 1,75 |
| 1000 | 6,02 | 0,78 | 0,31 | 91,02 | 1,86 |
| 1500 | 6,04 | 0,89 | 0,31 | 90,85 | 1,87 |
| 2000 | 6,03 | 1,02 | 0,28 | 90,65 | 2,03 |

Wie aus der Tabelle 2 zu entnehmen, wurden bei der Hydrierung von Roh-Isononanal am BaO-modifizerten Cu/Cr/Ni-Katalysator (aus Beispiel 2) im Vergleich zum Standardkatalysator (aus Beispiel 1) geringere Mengen an Hochsieder gebildet. Die C₉-Aldehyd-Restgehalte stiegen im Unterschied zum Standardkatalysator mit der Versuchszeit deutlich langsamer an, was auf einen geringeren Rückgang der Aktivität hindeutet. Die im Vergleich zu unmodifiziertem Standardkatalysator verbesserte Selektivität und Aktivität des zweiten Katalysators hatte zur Folge, dass die Ausbeuten am Wertprodukt Isononanol während der Versuchzeit nicht nennenswert gemindert wurde. Die hohen C₉-Alkohol-Gehalte von über 90,5 Gew.-% blieben auch nach 2000 Stunden erhalten.

## Patentansprüche

1. Verfahren zur Herstellung von Alkoholen durch Hydrierung von Carbonylverbindungen,
**dadurch gekennzeichnet,**
**dass** man die Hydrierung in Gegenwart eines Hydrierkatalysators bestehend aus einem Trägermaterial und zumindest einem hydrieraktiven Metall, wobei das Trägermaterial auf Titandioxid, Zirkondioxid, Aluminiumoxid, Siliziumoxid oder deren Mischoxide basiert und das hydrieraktive Metall mindestens ein Element der Gruppe Kupfer, Kobalt, Nickel, Chrom ist,
wobei
das Trägermaterial das Element Barium enthält, wobei der Gehalt an Bariumverbindung, berechnet als Bariumoxid, bezogen auf den reduzierten Katalysator, im Bereich von 0,1 bis 2 Massen-%, insbesondere im Bereich von 0,3 bis 0,7 Massen-% liegt.

2. Verfahren nach Anspruch 1,
**dadurch gekennzeichnet,**
**dass** mit Wasserstoff in einem Druckbereich von 5 bis 100 bar,
vorzugsweise von 5 bis 40 bar, besonders bevorzugt 10 bis 25 bar, und bei einer Hydriertemperatur von 120 bis 220 °C, vorzugsweise von 140 bis 190 °C, hydriert wird.

3. Verfahren nach Anspruch 1_oder 2,
**dadurch gekennzeichnet,**
**dass** als Carbonylverbindungen gesättigte oder ungesättigte Aldehyde oder Ketone mit4 bis 25 C-Atomen eingesetzt werden.

4. Verfahren nach den Ansprüchen 1_bis 3,
**dadurch gekennzeichnet,**
**dass** durch Hydroformylierung erhaltene Carbonylverbindungen eingesetzt
werden.

5. Verfahren nach den Ansprüchen 1_bis 4,
**dadurch gekennzeichnet,**
**dass** aus C₈- oder C₁₂-Olefinen oder C₈- oder C₁₂-Olefingemischen hergestellte Hydroformylierungsgemische eingesetzt werden.

6. Verfahren nach den Ansprüchen 1_bis 5,
**dadurch gekennzeichnet,**
**dass** der durch Hydroformylierung von Dibuten erhältliche C₉-Aldehyd Isononanal eingesetzt wird.

7. Verfahren nach den Ansprüchen 1_bis 3,
**dadurch gekennzeichnet,**
**dass** ein Decenalgemisch, hergestellt durch Kondensation von C₅-Aldehyden, wie insbesondere Valeraldehyd, eingesetzt wird.

## Claims

1. Process for preparing alcohols by hydrogenation of carbonyl compounds, **characterized in that** the hydrogenation is carried out in the presence of a hydrogenation catalyst which comprises a support material and at least one hydrogenation-active metal and in which the support material is based on titanium dioxide, zirconium dioxide, aluminium oxide, silicon oxide or mixed oxides thereof and the hydrogenation-active metal is at least one element from the group consisting of copper, cobalt, nickel, chromium, wherein the support material contains the element barium, where the content of barium compound, calculated as barium oxide and based on the reduced catalyst, is in the range from 0.1 to 2% by mass, in particular in the range from 0.3 to 0.7% by mass.

2. Process according to Claim 1, **characterized in that** the hydrogenation is carried out using hydrogen in a pressure range from 5 to 100 bar, preferably from 5 to 40 bar, particularly preferably from 10 to 25 bar, and at a hydrogenation temperature of from 120 to 220°C, preferably from 140 to 190°C.

3. Process according to Claim 1 or 2, **characterized in that** saturated or unsaturated aldehydes or ketones having from 4 to 25 carbon atoms are used as carbonyl compounds.

4. Process according to Claims 1 to 3, **characterized in that** carbonyl compounds obtained by hydroformylation are used.

5. Process according to Claims 1 to 4, **characterized in that** hydroformylation mixtures prepared from C₈-or C₁₂-olefins or C₈- or C₁₂-olefin mixtures are used.

6. Process according to Claims 1 to 5, **characterized in that** the C₉-aldehyde isononanal which can be obtained by hydroformylation of dibutene is used.

7. Process according to Claims 1 to 3, **characterized in that** a decenal mixture prepared by condensation of C₅-aldehydes, in particular valeraldehyde, is used.

## Revendications

1. Procédé de fabrication d'alcools par hydrogénation de composés carbonyle, **caractérisé en ce que** l'hydrogénation est réalisée en présence d'un catalyseur d'hydrogénation constitué par un matériau support et au moins un métal actif pour l'hydrogénation, le matériau support étant à base de dioxyde de titane, de dioxyde de zirconium, d'oxyde d'aluminium, d'oxyde de silicium ou leurs oxydes mixtes, et le métal actif pour l'hydrogénation étant au moins un élément du groupe constitué par le cuivre, le cobalt, le nickel, le chrome, le matériau support contenant l'élément baryum, la teneur en composé de baryum, calculée en tant qu'oxyde de baryum, par rapport au catalyseur réduit, se situant dans la plage allant de 0,1 à 2 % en masse, notamment dans la plage allant de 0,3 à 0,7 % en masse.

2. Procédé selon la revendication 1, **caractérisé en ce que** l'hydrogénation est réalisée avec de l'hydrogène dans une plage de pression allant de 5 à 100 bar, de préférence de 5 à 40 bar, de manière particulièrement préférée de 10 à 25 bar, et à une température d'hydrogénation de 120 à 220 °C, de préférence de 140 à 190 °C.

3. Procédé selon la revendication 1 ou 2, **caractérisé en ce que** des aldéhydes ou des cétones saturés ou insaturés contenant 4 à 25 atomes C sont utilisés en tant que composés carbonyle.

4. Procédé selon les revendications 1 à 3, **caractérisé en ce que** des composés carbonyle obtenus par hydroformylation sont utilisés.

5. Procédé selon les revendications 1 à 4, **caractérisé en ce que** des mélanges d'hydroformylation préparés à partir d'oléfines en C₈ ou C₁₂ ou de mélanges d'oléfines en C₈ ou C₁₂ sont utilisés.

6. Procédé selon les revendications 1 à 5, **caractérisé en ce que** l'aldéhyde en C₉ isononanal pouvant être obtenu par hydroformylation de dibutène est utilisé.

7. Procédé selon les revendications 1 à 3, **caractérisé en ce qu'**un mélange de décénals, préparé par condensation d'aldéhydes en C₅, tels que notamment le valéraldéhyde, est utilisé.
